# EUROPEAN PATENT APPLICATION

(11) **EP 4 198 141 A1**
(43) Date of publication of application: **21.06.2023**
(21) Application number: 21306766.3
(22) Date of filing: 14.12.2021
(51) Int. Cl.: C12Q 1/02, C12Q 1/04, C12Q 1/06

(54) **METHOD FOR DETECTING AND OPTIONALLY QUANTIFYING MICROORGANISMS**

(71) Applicant: C4Diagnostics, 13013 Marseille (FR)
(72) Inventor: FUGIER, Emilie, 13008 Marseille (FR)
(74) Representative: Santarelli

(57) **Abstract**

The present invention concerns a method for detecting and optionally quantifying microorganisms in a liquid sample, comprising the step of (a) passing the liquid sample through a membrane thereby depositing the potentially present microorganisms on the upper surface of said membrane, (b) putting the lower surface of said membrane into contact with a culture medium and incubating the whole under conditions suitable for promoting the formation of microcolonies from the microorganisms, (c) putting the lower surface of said membrane into contact with a fluorescent compound capable of labeling an endogenous constituent of the microorganisms in order to label potentially formed microcolonies, (d) washing the lower surface of said membrane thereby eliminating the excess of fluorescent compound, and (e) detecting and optionally quantifying the potentially formed microcolonies labeled with the fluorescent compound, wherein the detection is performed using an optical device having a magnification between 1 and 50 and an optical resolution comprised between 6-20 Mpix.

## Description

### TECHNICAL FIELD

The present invention belongs to the general field of microbiology and more particularly to the field of the detection of microorganisms present in a liquid sample.

The present invention proposes a method for detecting and optionally quantifying viable microorganisms present in a liquid sample. This method makes it possible to have a detection and optional quantification in a very short time, typically in less than 10 h in particular, in less than 9 h, and, more particularly, in less than 8 h without using a sophisticated and expensive equipment and without affecting the viability of the microorganisms which can be used after the implementation of the method, for further growth and/or further experiments such as, for example, for antibiotic susceptibility.

### STATE OF THE PRIOR ART

In the field of medicine, in the food, biotechnological and pharmaceutical industries, for military and civilian defense, and environmental control, it is very important to have rapid, simple and reliable identification of microorganisms.

Indeed, early detection and rapid identification of microorganisms in liquid samples are indispensable not only for successful decontamination of infected environments and products but also for successful treatment of patients with microbial infections. In particular, in the case of sepsis and other infections which are associated with high morbidity and mortality, there is a real need for a fast and reliable detection of microorganisms in biological fluids and of their susceptibility to antibiotics.

For example, bioburden testing, also known as "microbial limit testing", is performed on pharmaceutical products and medical products for quality control purposes. Products or components used in the pharmaceutical or medical field require control of microbial levels during processing and handling. Bioburden or microbial limit testing on these products proves that these requirements have been met. The aim of bioburden testing is to measure the total number of viable microorganisms (total microbial count) on a medical device prior to its final sterilization before implantation or use.

More specifically, in the field of diagnosis, urinary tract infection (UTI) is a disease that implies microbial analysis of a patient's urine.

UTI a general term for infectious diseases in various parts of the urinary system.

Symptoms from a lower urinary tract include pain with urination, frequent urination, and feeling the need to urinate despite having an empty bladder. Symptoms of a kidney infection include fever and flank pain usually in addition to the symptoms of a lower UTI. In some cases, the urine may appear bloody. In the very old and the very young, symptoms may be vague or non-specific. A common cause of infection is the presence of pathogenic *Escherichia coli* in the urinary tract, though other bacteria, viruses or fungi may be the cause.

UTI is a common infection that is usually treated by antibiotics and can result in serious complications, such as renal failure. In order to avoid these complications and overuse of antibiotics, efficient diagnosis and treatment is important. Rapid and safe diagnostic methods are thus required.

In both cases, analyses take usually 12 h to 36 h when using gold standard methods (plating on solid culture medium) and counting visible colonies after culture. This delay involves a subsequent delay in quality controls and also in therapeutics when applied to clinical and diagnostic, research and analysis.

Alternative methods have thus been proposed.

The International application WO 2013/107759 A1 proposes to use the click chemistry for identification of microorganisms **[1]**. More particularly, the bacteria are labeled by incorporating, into their membrane lipopolysaccharides (LPS), a modified sugar component bearing a bio-orthogonal chemical reporter (8-azido-3,8-dideoxy-D-manno-octulosonic acid (Kdo-N₃)), and enabling the detection thereof with a click chemistry reaction, allowing further detection of the chemical reporter such as a fluorescent compound (A488-yne). It should be noted that the click chemistry is subsequently lethal for the bacteria and the assimilation of the modified sugar by bacteria takes a long time. Moreover, the proposed method is specific for Gram negative bacteria since the 3-deoxy-D-manno-oct-2-ulosonic acid or ketodeoxyoctonic acid (Kdo) is only found in the LPS inner core of these bacteria and thus cannot be used to detect other microorganisms such as, for example, Gram positive bacteria such as illustrated in example 7 and yeasts.

The International application WO 2013/130875 A1 proposes a method for rapid determination of antibiotic susceptibility of a microorganism within hours after sample collection **[2]**. This method requires neither a prior isolation of the microorganisms by culture nor their prior identification. Indeed, in this method, the microorganisms are extracted from the test sample using a solid substrate coated with microbe-binding molecules such as beads, fibers, filters, screens, mesh, tubes, fluidic or microfluidic channels coated with opsonins, lectins, antibodies and antigen binding fragments thereof, proteins, peptides, nucleic acids, carbohydrates and/or lipids. Once extracted, the substrate-bound microorganisms are incubated in presence of antibiotic agent(s) and their sensitivity against the latter is determined by comparing the result with an incubation free of antibiotic agent. This method necessitates to use microbe-binding molecules presenting a broad spectrum in order to recover any microorganism present in the test sample.

Consequently, there is a need for a rapid, accurate and cost-effective method to detect and optionally quantify living microorganisms which overcomes the limitations of the prior art. In particular, the method according to the invention does not involve the use of an expensive optical device nor a PCR amplification.

### DISCUSSION OF THE INVENTION

The present invention proposes a method for detecting and optionally quantifying microorganisms present in a liquid sample which is performed on the microorganisms in the form of colonies and more particularly micro-colonies using a universal labeling i.e. a fluorescent compound and a membrane the upper and lower surfaces of which have clearly defined different functions. Indeed, the microorganisms are deposited on the upper surface and develop thereon in microcolonies, while the lower surface is the one in contact with the culture medium, the fluorescent compound and the washing solution(s).

Implementing the method according to the invention on colonies of microorganisms makes it possible to detect only living ones. Furthermore, the method does not affect the viability of the microorganisms which go on growing and can be used later for further characterization such as determination of antibiotics susceptibility. The viability is not affected in particular because no fixation is requested to scan the microorganisms labeled with the fluorescent compound. Another advantage of this lack of fixation is the fact that the steps of labeling with the fluorescent compound, of washing and of scanning can be repeated as many times as necessary.

Detection methods using a fluorescent compound are well-known in the art but, as already mentioned, a fixation step is requested and they are often used on microorganisms in the form of single cells with the fluorescent compound being deposited onto the microorganisms. The present invention implements microorganisms in the form of microcolonies on the upper surface of a membrane and a fluorescent compound put into contact with the microorganisms via the lower surface of this membrane. It should be noted that, before the present invention, it was not at all obvious that what was realized on single cells would work on microcolonies and that the fluorescent compound would be able, during the short contact time, to diffuse uniformly and to be sufficient for an observation without requesting sophisticated and expensive equipment.

In addition, thanks to the labeling of the microorganisms and the washing via the lower surface of the membrane, there is no risk of eliminating microorganism microcolonies and the fixation is not necessary.

More particularly, the present invention concerns a method for detecting and optionally quantifying microorganisms in a liquid sample, comprising the steps of:
a) passing the liquid sample through a membrane thereby depositing the potentially present microorganisms on the upper surface of said membrane,
b) putting the lower surface of said membrane into contact with a culture medium and incubating the whole under conditions suitable for promoting the formation of microcolonies from the microorganisms,
c) putting the lower surface of said membrane into contact with a fluorescent compound capable of labeling an endogenous constituent of the microorganisms in order to label potentially formed microcolonies,
d) washing the lower surface of said membrane thereby eliminating the excess of fluorescent compound, and
e) detecting and optionally quantifying the potentially formed microcolonies labeled with the fluorescent compound, wherein the detection is performed using an optical device having a magnification between 1 and 50 and an optical resolution comprised between 6-20 Mpix.

In the present application, all the ranges must be interpreted as limits included.

The present invention can be carried out on any liquid sample that may contain microorganisms by nature or following a contamination.

Typically, this liquid sample can be a biological fluid; a plant fluid such as sap, nectar and root exudate; a sample in a culture medium or in a biological culture reactor such as a cell culture of higher eukaryotes, of yeasts, of fungi or of algae; a liquid obtained from one (or more) animal or plant cell(s); a liquid sample obtained from an animal or plant tissue; a liquid sample obtained from a food matrix; a liquid sample from a chemical reactor; municipal, river, pond, lake, sea, or air-cooled tower water; a sample from a liquid industrial effluent; waste water coming in particular from intensive animal production or industries of the chemical, pharmaceutical, cosmetic or nuclear field; a liquid sample from a pharmaceutical product; a liquid sample from a cosmetic product; a fragrance; a soil sample or a mixture thereof.

Within the scope of the present invention, by "sample" is meant any type of sample collection, for example, by contact, scraping, drilling, draining, washing, rinsing, suction, pumping, etc ...

When the liquid sample is a biological fluid, the latter is advantageously selected from the group consisting of blood such as whole blood or anti-coagulated whole blood, blood serum, blood plasma, lymph, saliva, sputum, tears, sweat, sperm, urine, faeces, milk, cerebrospinal fluid, interstitial fluid, a fluid isolated from bone marrow, a mucus or fluid isolated from respiratory tract, intestinal tract or genito-urinary tract, cellular extract, tissue extract and organ extract. Thus, the biological fluid can be any fluid naturally secreted or excreted from a human or animal body or any fluid recovered, from a human or animal body, by any technique known to those skilled in the art such as extraction, sampling or washing. The steps of recovering and isolating these different fluids from the human or animal body are made prior to the implementation of the method according to the present invention. Preferably, the liquid sample is urine.

Likewise, if one of the contemplated samples does not allow implementing the method according to the present invention, for example because of its nature being in particular solid, its concentration or elements it contains such as solid residues, waste, suspension or interfering molecules, the sampling such as defined hereinafter further comprises a prior step of preparing the sample with optionally solubilizing the sample by techniques known to those skilled in the art such as filtration, precipitation, dilution, distillation, mixing, concentration, lysis, etc...

For example, when the liquid sample is urine, the latter may be filtrated in orderto remove interfering substances such as crystals, hyaline structure, cell aggregates and/or big eukaryotic cells. To this extend, a sterile sieving device with a porosity of 5 µm or 10 µm such as a pluriStrainer^{®} can be used. Nevertheless, this filtration is optional.

The potentially present microorganisms to be detected and optionally quantified by the method of the present invention are organisms and notably unicellular organisms of the eukaryotic type or organisms and notably unicellular organisms of the prokaryotic type.

The microorganisms of the eukaryotic type may be yeasts such as yeasts of the genus Saccharomyces or Candida like *Candida albicans*, fungi, algae or mixtures thereof.

The microorganisms of the prokaryotic type are bacteria which may be of the Gram positive type or Gram negative type, archaea or mixtures thereof.

Thus the potentially present microorganisms to be detected and optionally quantified by the method of the present invention are advantageously selected from yeasts, fungi, algae, bacteria of the Gram positive type, bacteria of the Gram negative type, archaea and mixtures thereof.

Among the bacteria, mention may be made, as an example and in a non-exhaustive way, of bacteria belonging to branches of spirochetes and chlamydiae, of bacteria belonging to the families or genera of Alcaligenaceae, Pasteurellaceae, Enterobacteriaceae, Enterococcus, Staphylococcus, Pseudomonadaceae, Streptococcus, Micrococci, Legionellaceae, Mycobacteria, Bacillaceae and Cyanobacteria. More particularly, these bacteria may be selected from the group consisting of *Bordetella pertussis, Haemophilus influenza, Citrobacter freundii, Escherichia coli, Enterococcus faecalis, Klebsiella pneumoniae, Klebsiella pneumoniae, Klebsiella aerogenes, Legionella pneumophila*, *Morganella morganii, Pseudomonas aeruginosa, Proteus mirabilis, Serratia marcescens*, *Bacillus anthracis*, *Staphylococcus aureus*, *Staphylococcus saprophyticus* and *Streptococcus agalactiae.*

Preferably, said microorganisms are *Escherichia coli*, *Pseudomonas*, *Candida* and/or *Staphylococcus.*

From among archaea, as examples and in a non-exhaustive way, mention may be made of the archaea belonging to the phyla of Crenarchaeotes and Euryarchaeotes.

In the step (a) of the method according to the present invention, the potentially present microorganisms are separated from the liquid part of the liquid sample containing them. To this extend, a membrane is used.

It is clear that the membrane implemented in the present invention is able to retain the potentially present microorganisms contained in the liquid sample and/or is impermeable to these microorganisms. Advantageously, this membrane is porous with naturally occurring pores or artificially created pores and with a porosity suitable for retaining the microorganisms potentially present or likely to be present in the liquid sample. Advantageously, this porosity is less than 0,5 µm and in particular of 0,45 µm or less.

Any membrane usually implemented for filtering a liquid sample and retaining the microorganisms present or likely to be present therein may be used within the present invention. Suitable membranes for use in the present invention include, without limitation, membranes made of polytetrafluoroethylene (PTFE), polyester, polycarbonate, nylon, polyvinylidene fluoride (PVDF), cellulose and cellulose derivatives, such as cellulose acetate, cellulose nitrate, regenerated cellulose, nitrocellulose, cellophane, or mixed cellulose esters (MCE). Such membranes are in particular available from the companies Whatman, Merck Millipore, and Sigma Aldrich.

Advantageously, the membrane implemented in the present invention is a MCE membrane, in particular a black MCE membrane and, more particularly, the black MCE membrane No. 1 sold by Merck Millipore.

It should be noted that the membrane implemented in the present invention do not have to be subjected to any pretreatment before step (a). This pretreatment may be a coating and in particular a coating with microbe-binding molecules as defined in **[2]**.

Following step (a) and before step (b), the lower surface of the membrane may be subjected to at least one rinsing in order to remove all traces of the liquid sample and/or contaminants such as inorganic contaminants present in this liquid sample following its preparation. These inorganic contaminants are, for example, inorganic residues such as borate residues from borate tube recovering urine. This rinsing is typically performed with an aqueous rinsing solution that does not affect the microorganisms present at the upper surface of the membrane. This solution can also help eliminate non-specific interactions. It can comprise one or more of the following components: a buffer such as a Tris, phosphate, acetate or borate buffer; salts such as KCI, NaCI, (NH₄)₂SO₄, MgCl₂ or CaCl₂; detergents or surfactants such as Tween^{®}, Triton^{®}, sodium dodecyl sulfate (or SDS) or bovine serum albumin (BSA). Typically, the rinsing is performed using a rinsing solution comprising phosphate buffered saline (or PBS) and 0.1% Tween^{®} and having a pH comprised between 7 and 7.5. In particular embodiments, this rinsing may be repeated at least twice, using, for each rinsing, an identical or different rinsing solution.

Typically, the duration of this optional washing/rinsing step is equal to or below 15 min, notably equal to or below 10 min, in particular, of 5 min (i.e. 5 min ± 3 min).

This optional washing/rinsing step is performed at a temperature comprised between may be carried out at a temperature of between 10°C and 40°C, advantageously between 15°C and 30°C and in particular at room temperature (i.e. 23°C ± 5°C).

The step (b) of the method according to the present invention is a culturing step in order to form microcolonies from the microorganisms in the form of single cells deposited onto the upper surface of the membrane obtained after step (a).

The term "microcolony" is to be understood a colony not visible to the naked eye. Typically, a microcolony comprises between has a diameter less than 100µm, preferably between 10 and 100µm, more preferably between 25 and 100 µm, still more preferably 40 and 100 µm and even still more preferably between 50 and 100µm such as between 50 and 80 µm.

In other words, during step (b) of the method according to the present invention, the microorganisms are maintained in an environment that allows them to increase in number through cell division in order to form microcolonies. This environment includes not only the culture medium put into contact with the lower surface of the membrane but also other conditions such as a temperature and an atmosphere that permit cell growth. These conditions are defined as "conditions suitable for promoting the formation of microcolonies from the microorganisms". Typically, the temperature implemented during step (b) is comprised between 25°C and 40°C. Particular examples of temperature implemented at step (b) are 30°C, 32°C, 37°C and 39°C. Typically, concerning the atmospheric conditions, the step (b) may be performed under air, under air + 5% CO₂ or under a gaseous mixture consisting of 5% O₂, 5% CO₂ and 90% N₂.

In a first embodiment, the culture medium implemented at step (b) of the method according to the present invention is a liquid culture medium.

This liquid culture medium implemented at step (b) of the method according to the present invention may be any liquid medium suitable for culturing microorganisms, known by those skilled in the art. The latter has a carbon source, such as glucose or glycerol; a nitrogen source, such as ammonium or nitrate or amino acids; and salts and/or trace elements and vitamins for the growth of microorganisms. In particular, this liquid culture medium may not be selective to a particular type of microorganisms. Alternatively, it may be selective to a particular type of microorganisms.

As examples of liquid media suitable for using at step (b) of the method according to the present invention, one can cite Nutrient Broth No. 1 (NB1), Nutrient Broth No. 2 (NB2), Mueller Hinton broth, Lysogenic Broth (LB) (also known as Luria Bertani), Tryptic SoyBean (TSB) culture liquid and Brain Heart Infusion (BHI) broth. Such culture media are in particular available from the companies ThermoFisher, Bio-Rad Laboratories and Sigma Aldrich.

These different liquid culture media may be supplemented by one or more elements selected in the group consisting of an antioxidant agent such as sodium pyruvate, glutamate, calcium chloride and magnesium chloride; a multi-vitamin such as Polyvitex^{®} (PVX); a source of iron such as iron citrate or Vitox^{®}; a protein supplement such as Bovine Serum Albumin (BSA); and an anti-clumping agent such as citrate salt.

Advantageously, the liquid culture medium is sterile or has been sterilized before contacting the lower surface of the membrane at step (b) of the method according to the present invention. The culture medium can be sterilized by various techniques known in the art, such as, but not limited to, the use of aseptic techniques to autoclave and/or prepare the culture medium.

In a second embodiment, the culture medium implemented at step (b) of the method according to the present invention is a solid culture medium.

In this second embodiment, the solid culture medium may be a diffusion intermediate impregnated with a liquid culture medium such as previously defined. A diffusion intermediate is a solid support which has to be porous in order to allow good impregnation with the liquid culture medium such as previously defined. Advantageously, the diffusion intermediate is a porous solid support such as a pad in a material selected from the group consisting of paper notably of a cellulose nature; cotton paper; agarose; gelatin; cellulose; methylcellulose; carboxymethylcellulose; nitrocellulose; cellulose acetate ester; an alginate; a polyolefin; a porous membrane and notably an ion exchange porous membrane; a resin of the sephadex type packaged as a membrane or a membrane of a perfluorinated polymer such as PVDF; a felt fabric; a glass fiber fabric; a membrane in an organic polymer such as polyethylene, polypropylene or mixtures thereof; a nylon fabric; a polyacrylamide gel; a sepharose gel and one of their mixtures.

As a variant of this second embodiment, the solid culture medium may be a nutritive gel. The latter may be any nutritive gel suitable for culturing microorganisms, known by those skilled in the art. As for liquid culture medium, this nutritive gel has a carbon source, such as glucose or glycerol; a nitrogen source, such as ammonium or nitrate or amino acids; and salts and/or trace elements and vitamins for the growth of microorganisms. In particular, this solid culture medium may not be selective to a particular type of microorganisms. Alternatively, it may be selective to a particular type of microorganisms.

As examples of solid media suitable for using at step (b) of the method according to the present invention, one can cite nutrient agar, Tryptic Soy Agar (TSA), Mueller Hinton agar, Legionella Glycine Vancomycine Polymyxine Cycloheximide (GVPC) agar, buffered charcoal yeast extract (BCYE) agar, Buffered cefaMandole Polymyxine Anisomycine α-cetoglutarate (BMPAα) agar, Reasoner's 2A (R2A) agar, Sabouraud Dextrose Agar (SDA), Columbia agar with horse blood, blood agar such as sheep blood agar, chocolate agar with Vitox, Haemophilus Test Medium (HTM) agar and charcoal agar. Such culture media are in particular available from the companies ThermoFisher, Oxoid and VWR.

Advantageously, when the culture medium implemented at step (b) of the present invention is solid, it is blood agar.

In addition, it is possible, when using a solid culture medium, to add some liquid culture medium such as previously defined between the solid culture medium and the membrane, on the upper surface of which are the microorganisms. Nevertheless, this addition is optional.

Typically, depending on the type of microorganisms present in the liquid sample, the step (b) has to last no more than 10 h, notably no more than 9 h, in particular no more than 8 h and more particularly no more than 7 h. Indeed, as for example, from the results obtained by the inventors, in particular, in the conditions as disclosed in the hereinafter experimental part, microcolonies of *E. coli*, of *E. faecalis*, or of *C. albicans* can be detected after 6 h of incubation with culture medium and microcolonies of *P. aeruginosa* or of *S*. *saprophyticus* after 8 h of incubation.

The step (c) of the method according to the present invention is a labeling step in order to label with a fluorescent compound the microcolonies potentially formed onto the upper surface of the membrane obtained after step (b).

Generally, the term "fluorescent compound" refers to a compound capable of emitting light when excited by another light of appropriate wavelength. The term "fluorescent", as applied to a compound, can be used to refer to its property of absorbing energy (such as UV, visible or IR radiation) and re-emitting at least a fraction of that energy as light over time.

The present invention implements a fluorescent compound capable of recognizing and binding an endogenous constituent of the microorganisms. By definition, an endogenous constituent means a constituent that originates from within the microorganism. Thus the process according to the present invention is clearly different from the one disclosed in the International application WO 2013/107759 **[1]** because in this document, the fluorescent compound such as A488-yne recognizes an element (Kdo-N₃) which is not an endogenous constituent of Gram negative bacteria, contrary to Kdo.

Typically, the fluorescent compound implemented at step (c) of the method according to the present invention is capable to recognize and bind, and thus to label, mitochondria, cell membranes or nucleic acids.

In a first embodiment, the fluorescent compound implemented at step (c) of the method according to the present invention is capable to recognize and bind, and thus to label, mitochondria. Advantageously, this fluorescent compound is selected from the group consisting of Rhodamine123, DiOC₆(3), DiOC₇(3), JC-1, MitoTracker Orange and Red CMXRos.

In a second embodiment, the fluorescent compound implemented at step (c) of the method according to the present invention is capable to recognize and bind and, thus to label, cell membranes. Advantageously, this fluorescent compound is selected from the group consisting of FM1-43 and FM4-64.

In a third embodiment, the fluorescent compound implemented at step (c) of the method according to the present invention is capable to recognize and bind, and thus to label, nucleic acids. "Nucleic acid" is understood to mean a single-stranded or double-stranded desoxyribonucleic acid (DNA), a ribonucleic acid (RNA) such as a messenger RNA or a ribosomal RNA.

The fluorescent compounds specific for nucleic acids are in particular selected from the fluorescent intercalating agents, dyes binding to the bases A:T or the bases G:C and the permeating or non-permeating cyanines. More particularly, said fluorescent compounds are selected from the group consisting of ethidium bromide, thiazole orange, thiazole blue and derivatives thereof, thioflavin S, thioflavin T, thioflavin TCN, diethylquinolylthio-cyanine iodide (DEQTC), TOTO1, TOPRO1, TOTO3, TOPRO3, YOYO1, Hoechst 33258, Hoechst 33342, Hoechst 34580, 4',6-diamidino-2-phenylindole (DAPI), pyronin Y, acridine orange, auramine O, calcein, olamin-O, Oxazine 750, Astra blue and the SYTO series comprising in particular SYTO 11, SYTO 12, SYTO 13, SYTO 15, SYTO 16, SYTO 18, SYTO 62, SYTO 80 or SYTO 81.

Preferably, the fluorescent compound is not toxic for the microorganisms and its detection method does not affect the microorganism's viability.

More preferably, the fluorescent compound is selected in the group consisting of 4',6-diamidino-2-phenylindole (DAPI), Rhodamine123, DiOC₆(3), DiOC₇(3), JC-1, MitoTracker Orange, Red CMXRos, FM1-43, FM4-64, thiazole orange, thiazole blue and derivatives thereof, TOTO1, TOPRO1, TOTO3, TOPRO3, YOYO1, Hoechst 33258, Hoechst 33342, Hoechst 34580, acridine orange, calcein, olamin-O, Oxazine 750, Astra blue and the SYTO series comprising in particular SYTO 11, SYTO 12, SYTO 13, SYTO 15, SYTO 16, SYTO 18, SYTO 62, SYTO 80 or SYTO 81.

In a particular embodiment, the fluorescent compound implemented at step (c) of the method according to the present invention is 4',6-diamidino-2-phenylindole (DAPI).

During this step (c), the fluorescent compound is put into contact with the lower surface of the membrane which thus is to be permeable thereto.

In a particular embodiment, the lower surface of the membrane may be brought into contact with a solution or dispersion of the fluorescent compound. The fluorescent compound is present in the solution or dispersion in an excess with respect to the microorganisms that are present on the upper surface of the membrane.

In another particular embodiment, the lower surface of the membrane may be brought into contact with a solution or dispersion of the fluorescent compound such as previously defined via a diffusion intermediate impregnated with said solution or dispersion. A diffusion intermediate is as previously defined for the solid culture medium provided that, in step (c), this diffusion intermediate is a solid support which has to be porous in order to allow good impregnation with the solution or dispersion of the fluorescent compound. A particular example of such a diffusion intermediate useful in step (c) is a glass fiber fabric impregnated with a solution or dispersion of a fluorescent compound such as a DAPI solution and more particularly a solution comprising 5 µg/ml of DAPI.

Typically, the duration of step (c) of the method according to the present invention is equal to or below 30 min, advantageously equal to or below 25 min, notably equal to or below 20 min, in particular, equal to or below 15 min and more particularly of 10 min (i.e. 10 min ± 2 min).

The step (c) of the method according to the present invention is performed at a temperature comprised between may be carried out at a temperature of between 10°C and 40°C, advantageously between 15°C and 30°C and more particularly at room temperature (i.e. 23°C ± 5°C).

In particular, the step (c) of the method according to the present invention is performed in the dark.

The step (d) of the method according to the present invention is a washing step in order to eliminate any fluorescent compound present at the level of the lower surface of the membrane. Indeed, the presence of this fluorescent compound adsorbed on the lower surface of the membrane might generate a background noise during the step (e).

The lower surface of the membrane is subjected to at least one rinsing in order to remove all traces of the fluorescent compound and/or of the solution or dispersion containing it. This rinsing is typically performed with a rinsing solution and, in particular, an aqueous rinsing solution that does not affect the microcolonies potentially formed on the upper surface of the membrane. This solution may also help eliminate non-specific interactions. It may comprise one or more of the following components: a buffer such as a Tris, phosphate, acetate or borate buffer; salts such as KCI, NaCl, (NH₄)₂SO₄, MgCl₂ or CaCl₂; and detergents or surfactants such as Tween^{®}, Triton^{®}, sodium dodecyl sulfate (or SDS) or serum bovine albumin (BSA). This rinsing may be repeated twice, three times, five times, 10 times and even 50 times using, for each rinsing, an identical or different rinsing solution. Typically, the rinsing is repeated three times with a solution comprising phosphate buffered saline (or PBS) and 0.1% Tween^{®} and having a pH comprised between 7 and 7.5.

Advantageously, this washing step (d) may be performed by continuously flowing the rinsing solution at the level of the lower surface of the membrane. This may be implemented using a washing device for membranes well-known in the field such as the washing module sold by DIAMIDEX^{®}.

Typically, the duration of step (d) of the method according to the present invention is equal to or below 40 min, advantageously equal to or below 30 min, notably equal to or below 20 min, in particular, of 15 min (i.e. 15 min ± 3 min).

The step (d) of the method according to the present invention is performed at a temperature comprised between may be carried out at a temperature of between 10°C and 40°C, advantageously between 15°C and 30°C and in particular at room temperature (i.e. 23°C ± 5°C).

In particular, the step (d) of the method according to the present invention is performed in the dark.

The step (e) of the method according to the present invention consists in placing the membrane obtained after step (d) under an optical device such as a microscope stage where it can be interrogated by an excitation source adapted to the fluorescent compound implemented.

Since the potentially formed microcolonies are not fixed for step (e) and no step of the method according to the present invention affects the viability of the microorganisms, the microorganisms of the potentially formed microcolonies are still alive after step (e).

Typically, the excitation source results in the excitation of the fluorescent compound labeling the microcolonies, followed by measurement of the emission of fluorescence from this compound.

The membrane can be manually manipulated to position microcolonies for interrogation, either by moving the membrane itself or moving the optical device on which the membrane is placed. Alternatively, the optical device is automatically controlled such that the membrane on the stage can be scanned.

Light sources capable of emission in the ultraviolet, visible and/or near-infrared spectra well-known to those skilled in the art may be used in the present invention. For example, light sources may be continuum lamps such as a deuterium or xenon arc lamp for generation of ultraviolet light and a tungsten halogen lamp for generation of visible/near-infrared excitation. Alternatively, the light source can be one or several LED(s). These light sources provide a broad emission range and the spectral bandwidth for specific excitation wavelengths may be reduced using filters such as optical interference filters, prisms and/or optical gratings.

The one skilled in the art will know which excitation source to be used depending on the fluorescent compound implemented in the method according to the present invention. Advantageously, the excitation source implemented at step (e) of the method according to the present invention is an optical microscope equipped with filters such as UV filter or Cy5 filter.

The measurement data obtained while performing step (e) may be analyzed by algorithms and in particular compared with control measurements obtained with microcolonies of known microorganisms. This comparison thus allows for correlation of measurement data obtained while performing step (e) with characterization of the microorganisms present in the liquid sample tested using various mathematical methods known to those skilled in the art.

Typically, the duration of step (e) of the method according to the present invention is equal to or below 20 min, advantageously equal to or below 15 min, notably equal to or below 10 min, in particular, of 7 min (i.e. 7 min ± 2 min).

Taking into account of the duration of the different steps of the method according to the present invention, the latter is performed in less than 10 h in particular, in less than 9 h, and, more particularly, in less than 8 h.

The optical device involved in step (e) of the present invention is a simple inexpensive optical device.

In a preferred embodiment of the invention, the method for detecting and optionally quantifying microorganisms in a liquid sample, comprises the steps of:
a) passing the liquid sample through a membrane thereby depositing the potentially present microorganisms on the upper surface of said membrane,
b) putting the lower surface of said membrane into contact with a culture medium and incubating the whole under conditions suitable for promoting the formation of microcolonies from the microorganisms,
c) putting the lower surface of said membrane into contact with a fluorescent compound capable of labeling an endogenous constituent of the microorganisms in order to label potentially formed microcolonies,
d) washing the lower surface of said membrane thereby eliminating the excess of fluorescent compound, and
e) detecting and optionally quantifying the potentially formed microcolonies labeled with the fluorescent compound, wherein the detection is performed using an optical device having a magnification between 1 and 10 and an optical resolution comprised between 8-17 Mpix.

Preferably, this optical device has a magnification between 1 and 8, more preferably between 2 and 4.

Preferably, the optical resolution of the optical device is comprised between 9 and 15, more preferably, between 11-13 Mpix.

In this preferred embodiment, steps (a)-(e) are as detailed above.

Then, as already explained, since the microcolonies are not fixed for step (e) of the method according to the present invention, steps (c), (b) and (e) may thus be repeated at least once. In other word, after a first step (e), steps (c), (b) and (e) may be repeated at least once, at least twice and advantageously several times. When steps (c), (b) and (e) are repeated several times, the repetition may be performed at time intervals that are regular or not. This makes it possible to follow the growth of different microorganisms present on the membrane, which may reach the microcolony stage at different times and/or to avoid false negative.

However, in a preferred embodiment, steps (c), (b) and (e) are not repeated and the method according to the invention comprises a single step (e) of detection and optionally quantification of the potentially formed microcolonies labeled with the fluorescent compound.

Advantageously, the method according the present invention further comprises the steps of:
f) putting the lower surface of the membrane comprising at least a labeled microcolony, into contact with a culture medium and incubating the whole under conditions suitable for promoting the formation of colonies from the microcolonies in order to form at least one colony,
g) identifying the microorganism of the formed colony, and
h) submitting the formed colony to an antibiotic susceptibility testing.

Thus, during step (f) of the method according to the present invention, the microcolonies are maintained in an environment that allows them to increase in number through cell division in order to form colonies. This environment includes not only the culture medium put into contact with the lower surface of the membrane but also other conditions such as a temperature and an atmosphere that permit cell growth. These conditions are defined as "conditions suitable for promoting the formation of colonies from the microcolonies". Typically, the temperature implemented during step (f) is comprised between 25°C and 40°C. Particular examples of temperature implemented at step (f) are 30°C, 32°C, 37°C and 39°C. Typically, concerning the atmospheric conditions, the step (f) may be performed under air, under air + 5% CO₂ or under a gaseous mixture consisting of 5% O₂, 5% CO₂ and 90% N₂.

The culture medium implemented at step (f) of the method according to the present invention is such as mentioned previously for step (b).

Typically, depending on the type of microorganisms present in the liquid sample, the step (f) has to last no more than 16 h, notably no more than 10 h, in particular no more than 8 h and more particularly no more than 7 h.

The method to be implemented for step (g) for identifying the microorganism of the formed colony is well-known in the art. Such identification is nowadays usually performed using mass spectrometry.

For step (h), several methods, also well-known in the art, exist. One is based on exposing microorganisms to antibiotics and uses dilution in broth. Another one involves selecting a strain of microorganisms, placing it on a agar plate and observing microorganism growth near antibiotic-impregnated discs (disc diffusion method). Still another one involves the use of strips impregnated with a gradient of antibiotics (Etest).

Other characteristics and advantages of the present invention will additionally be apparent to the one skilled in the art on reading the examples below, which are given as an illustration and not a limitation, with reference to the attached figures.

### SHORT DESCRIPTION OF THE DRAWINGS

Figure 1 is a schematic representation of the different steps of the method according to the present invention.

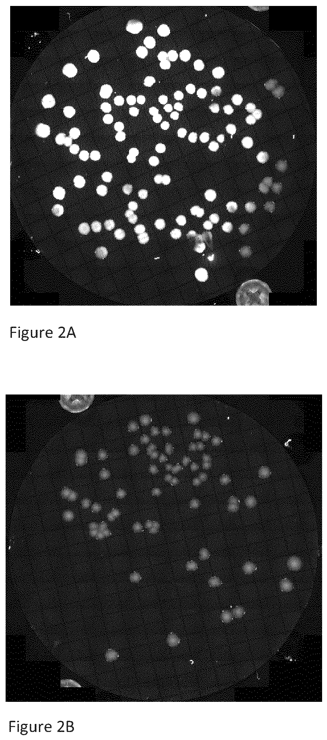

Figure 2 is a photography of the membrane obtained after implementation of the method according to the present invention using, as fluorescent compound, DAPI (Figure 2A) or Hoechst 33342 (Figure 2B).

### DETAILED DISCUSSION OF PARTICULAR EMBODIMENTS

### I. Use of the method according to present invention for detection UTI

### A. Material & methods

### Samples

Under the present comparative study, an external laboratory (Synlab) provided with fresh urinary samples every day (<24h) in boronated tubes (which inhibit bacterial growth, i.e. they are bacteriostatic) and which may be stored up to 48 hours at ambient temperature (AT).

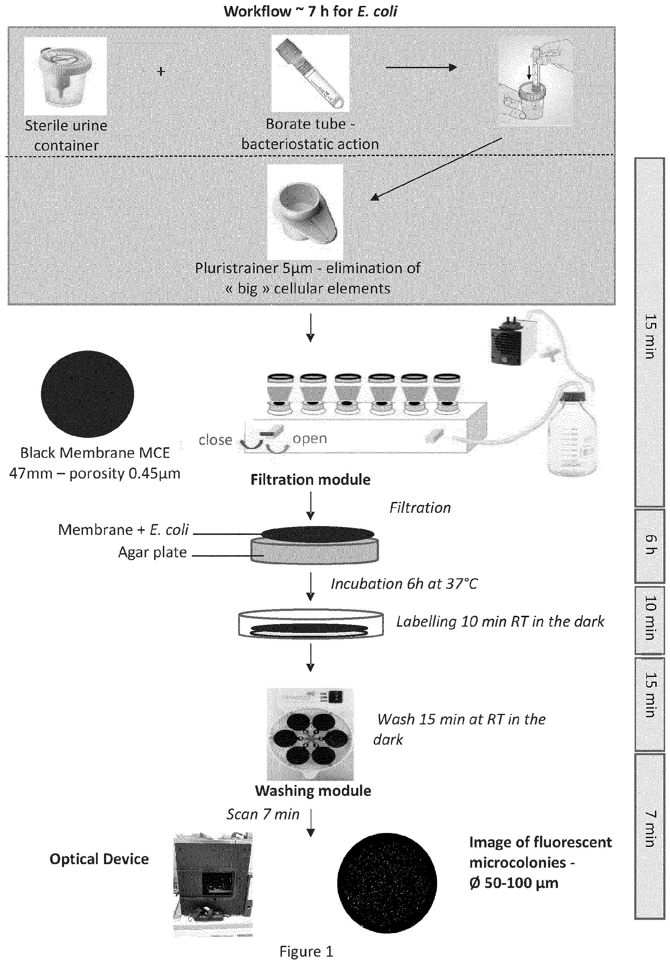

Next, the samples are treated as explained in Figure 1 and according to the protocol set out below.

### Description of resources and equipment used

### Human resources:

With this manual system and the current incubation times of 8 hours, 2 persons are required to treat 12 samples (up to 18 maximum) in a single day (10h for 12 samples and 11h for 18 samples).

### Equipment:

The filter bench operates with a vacuum pump and can filter 6 samples in parallel, and we have 18 reusable teflon filter units.

The rinsing bench operates continuously with a pump and enables the simultaneous washing of 6 samples. This washing is performed in series, the wash soluting flowing between the 6 samples and being recycled.

The optical bench enables 120 images to be taken using a plate system that scans the membrane surface bearing the microcolonies. It is equipped with LEDs and filters enabling the reading of blue fluorescence ("DAPI" channel) or red fluorescence ("FM4-64" channel).

### J-1: Preparation of the urine samples received from the external laboratory

1. Pre-filter 2 mL of received urine with the 5 µm cell screen and collect the filtrate in a 50 mL flask
2. Sead 10µL for filtered urine on a blood agar growth medium and set to incubate at 37°C for 16h in order to compare the colony forming units (CFUs) in the dish with the count made using the microcolonies counter.
3. Store the filtered urine at ambient temperature for D0 processing.

### D0 : Dilution of the urine samples

### Tneg and Tpos :

4. Tneg: 5 mL of phosphate buffer (=diluant)
5. Tpos (Eco BO52 dilution based on stock glycerol at -80°C):
   - Remove one Eppendorf tube/aliquot from the freezer at -80°C
   - Add 900 µL of phosphate buffer (0.05M pH7.4) in the tube and perform cascade dilution in steps of 10 to attain 10-6.
   - Add 30 µL of the 10-6 diluted solution to 5 mL of phosphate buffer in a 15 mL flask
   - Spread 30 µL of the 10-6 diluted solution on blood agar growth medium in order to obtain the CFUs therefrom.

### D0: Filtration of the urine samples

6. Dilute 10 µL of pre-filtered urine in 5 mL of phosphate buffer in a 15 mL bottle
7. Filter the Tneg and Tpos and the urine samples on reversed black MCE membranes (HABG047S6, Ø47mm) with the filtration bench (DIAMIDEX).
8. Deposit the membrane on blood agar (pre-warmed to 37°C in an incubator).
9. Incubate at 37°C for 8h

### Marking:

10. Prepare 1mL/sample of 1µg/ml DAPI solution (stock at1 mg/mL → use: 1µL/1 mL; stock -20°C) in phosphate buffer
11. Filter the DAPI solution at 0.22 µm
12. Incubate for 10 min at room temperature on a glass fiber pad (GE Healthcare Whatman: GF/A ref. 1820-047; batch 17076495)
13. Wash at the rinsing bench (Diamidex) with 500 mL PBS + 0.5mL tween20 (cfinal = 0.1%) (pre-warmed to 37°C) for 15 min The membrane may be stored at 4°C after marking until the following day
14. Reading on the optical bench (Diamidex "model 2", magnification: 4 and resolution: 12Mpix) is performed using the following parameters:
   ^{∗} UV power 10%
   ^{∗} exposure time: 262 ms
   ^{∗} Gain = 1.

Reconstitution as a mosaic of the 120 individual images is carried out automatically after the scan.

### Image Processing

The analysis of the 120 individual images is currently partly automatic thanks to two macros written in Fiji (ImageJ with plug-ins):

### 1) MacroOpenStackTiff.ijm:

- Open the 50 images of interest (i.e. of the membrane without the black borders)
- Assemble in a stack and save in ^{∗}.tiff format in a file named "Stack"

### 2) Macro_area_1.0.ijm:

- Parameters to input by the user prior to analysis using the algorithm:
   ^{∗} Minimum area (750 pixel²) and maximum area (250000 pixel²)
   ^{∗} The minimum circularity (0.6) (circle = perfectly round = 1)
- Output data:
   ^{∗} The position of the detected objects (ROI= "region of interest" to save in a zipped file)
   ^{∗} The area of the colonies in pixel2
   ^{∗} The degree for fluorescence (average, minimum and maximum)

### Analysis of the results

For the analysis of the data obtained from C4Diagnostics or from Synlab, the results of spreading on agar, which are considered as the control and the "true" result, are compared with the results of the scans on the optical bench after 8h incubation.

The sensitivity is calculated by dividing the number of false negatives (FN) by the total number of positive samples (refer also to the crossed classification such as that illustrated below in Table 1).

The specificity is calculated by dividing the number of false positives (FP) by the total number of negative samples.

These two values are expressed in %.

**Table 1: Crossed classification for a screening instrument (source : https://qastack.fr/stats/)**

| True diagnosis | | | | |
|---|---|---|---|---|
| Screening test | | Positive | Negative | Total |
| | Positive | α | b | a+b |
| | Negative | c | *d* | *c + d* |
| | Total | a+c | b + d | *N* |

For a statistical analysis more suited to in vitro diagnostic tests the NPV (negative predictive value; d / (c + d)) and the PPV (positive predictive value; a / (a + b)) are often used The NPV expresses the probability of patients with negative test results being correctly diagnosed (as negative). The NPV expresses the probability of patients with positive test results being correctly diagnosed (as positive). For these calculations, the site https://www.easycalculation.com/statistics/negative-predictive-value.php was used on which a crossed classification must be filled in and the calculation is then performed automatically.

### B. Results

Result are given in table 2 below.

**Table 2: Comparison of the results obtained by culture on a petri dish with a Columbia medium vs. method according to the invention.**

| | **Culture on Columbia medium** | **Method according to the invention** | | | **Comparison** |
|---|---|---|---|---|---|
| **Sample** | **POS/NEG** | **POS/NEG** | **CFU/mL** | **growth 24h** | |
| M001 | + | + | 2^{∗}10^5 | V | V |
| M002 | + | + | tapis | V | V |
| M003 | + | + | 3^{∗}10^4 | V | V |
| M004 | - | - | 600 | V | V |
| M005 | + | + | 800 | V | V |
| M006 | - | - | 0 | V | V |
| M007 | - | - | 0 | V | V |
| M008 | - | - | 200 | V | V |
| M009 | - | - | 300 | V | V |
| M010 | - | - | 0 | V | V |
| M011 | + | + | 2^{∗}10^4 | V | V |
| M012 | + | + | 3^{∗}10^3 | V | V |
| M013 | - | - | 0 | V | V |
| M014 | - (400) | - | 200 | V | V |
| M015 | + | + | > 10^5 | V | V |
| M016 | - | - | 0 | V | V |
| M017 | - | - | 0 | V | V |
| M018 | - | - | 0 | V | V |
| M019 | + | + | 4^{∗}10^3 | V | V |
| M020 | - | - | 0 | V | V |
| M021 | + | + | 3^{∗}10^3 | V | V |
| M022 | + | + | > 10^5 | V | V |
| M023 | + | + | > 10^5 | V | V |
| M024 | + | + | 10^5 | V | V |
| M025 | + | + | > 10^5 | V | V |
| M026 | - | - | 0 | V | V |
| M027 | + | + | > 10^5 | V | V |
| M028 | + | + | 10^4 | V | V |
| M029 | - | - | 0 | V | V |
| M030 | - | - | 300 | V | V |
| M031 | - | - | 300 | V | V |
| M032 | + | + | 10^4 | V | V |
| M033 | + | + | 10^3 | V | V |
| M034 | + | + | 2^{∗}10^3 | V | V |
| M035 | + | + | 4^{∗}10^3 | V | V |
| M036 | - | - | 0 | V | V |
| M037 | - | - | 300 | V | V |
| M038 | + | + | 4^{∗}10^4 | V | V |
| M039 | - | - | 0 | V | V |
| M040 | + | + | > 10^5 | V | V |
| M041 | - | - | 0 | V | V |
| M042 | + | + | 6,5 ^{∗} 10^3 | V | V |
| M043 | + | + | > 10^5 | V | V |
| M044 | - | - | 0 | V | V |
| M045 | - | - | 0 | V | V |
| M046 | + | + | 15 | X (5) | X |
| M047 | + | + | 4^{∗}10^3 | V | V |
| M048 | + | + | 3^{∗}10^3 | V | V |
| M049 | - | - | 500 | V | V |
| M050 | + | + | 6^{∗}10^3 | V | V |
| M051 | + | + | 4^{∗}10^4 | V | V |
| M052 | - | + | 3^{∗}10^3 | V | X |
| M053 | + | + | 6^{∗}10^3 | V | V |
| M054 | + | + | 3^{∗}10^3 | V | V |
| M055 | + | + | >10^5 | V | V |
| M056 | + | + | 3^{∗}10^4 | V | V |
| M057 | + | + | >10^5 | V | V |
| M058 | + | + | 4^{∗}10^4 | V | V |
| M059 | + | + | >10^5 | V | V |
| M060 | - | - | 500 | V | V |
| M061 | - | - | 100 | V | V |
| M062 | + | + | 10^3 | V | V |
| M063 | + | + | 10^4 | V | V |
| M064 | + | + | >10^5 | V | V |
| M065 | - | - | 500 | V | V |
| M066 | + | + | 4^{∗}10^4 | V | V |
| M067 | + | + | 10^4 | V | V |
| M068 | - | - | 0 | V | V |
| M069 | + | + | 2^{∗}10^4 | V | V |
| M070 | + | + | 3^{∗}10^3 | V | V |
| M071 | + | + | 8^{∗}10^3 | V | V |
| M072 | + | + | 3^{∗}10^3 | V | V |
| M073 | - | - | 300 | V | V |
| M074 | - | - | 0 | V | V |
| M075 | + | + | 700 | V | V |
| M076 | + | + | >10^5 | V | V |
| M077 | + | + | >10^4 | V | V |
| M078 | + | + | 2^{∗}10^3 | V | V |
| M079 | + | + | 2^{∗}10^3 | V | V |
| M080 | + | + | >10^5 | V | V |
| M081 | + | + | 4^{∗}10^4 | V | V |
| M082 | + | + | 2^{∗}10^3 | V | V |
| M083 | - | - | 400 | V | V |
| M084 | + | + | 5^{∗}10^4 | V | V |
| M085 | + | + | >10^5 | V | V |
| M086 | + | - | 600 | V | X |
| M087 | - | - | 0 | V | V |
| M088 | + | - | 100 | V | X |
| M089 | + | + | 10^3 | V | V |
| M090 | - | - | 0 | V | V |
| M091 | - | - | 0 | V | V |
| M092 | + | - | 600 | V | LOD |
| M093 | + | + | 10^3 | V | V |
| M094 | + | + | 10^3 | V | V |
| M095 | - | - | 300 | V | V |
| M096 | - | - | 100 | V | V |
| M097 | - | - | 0 | V | V |
| M098 | + | + | 10^4 | V | V |
| M099 | + | + | 10^3 | V | V |
| M100 | + | + | 10^3 | V | V |
| **M101** | **-** | **-** | 100 | v | V |
| **M102** | **+** | **+** | >10^5 | V | V |
| **M103** | **-** | **+** | 700 | V | LOD |
| **M104** | **+** | **+** | 6^{∗}10^3 | 10^4 | V |

| | | | | | |
|---|---|---|---|---|---|
| V: valid (check ok) X: not valid LOD: limit of detection | | | | | |

Taking into account the positives, negatives and the false positives and false negatives, the sensitivity and specificity (Table 3A) as well as the NPV and the PPV (Table 3B) of the test were calculated after crossed classification in Table 1.

**Table 3: The sensitivity and the Specificity (A) as well as the NPV and the PPV (B) of the test.**

| (A) | | Sensitivity | Specificity |
|---|---|---|---|
| | Total | 96,9% | 92,5% |

| (B) | | Total | |
|---|---|---|---|
| | | NPV | 95,0% |
| | | PPV | 95,0% |

### C. Conclusion

The present invention is a fast screening test for urinary infections, which allows to spread on agar only the urine of patients having bacteriuria considered as positive (≥ 10^3 CFU/ml). This makes it possible to reduce the operating and costs and consumption of reagents for test laboratories. Indeed, more than 70% of urinary cytobacteriological examinations are negative, with no germ being present in the urine sample analyzed. The other advantage of obtaining very fast screening of urinary infections is to reduce the unnecessary administration of antibiotics which would otherwise be given preventively, before obtaining the final bacteriuria threshold. It is such practice, initially directed to providing treatment as soon as possible, which has widely contributed to the increase in development of so-called antibiotic resistance.

### II. Use of different fluorescent compounds implemented in the method according to the present invention.

The protocol performed is similar to those described in Example I and illustrated at Figure 1:
First, urine was filtered onto a mixed ester cellulose black or white membrane, 45 mm and 0.45 µm of porosity. The filtration occurred onto a filter bench - 6 positions ("rampe de filtration - 6 positions"). Then the membrane is washed once with 5mL of Phosphate Buffer 0.05M pH7.2 again by filtration (to remove borate residues from borate tube recovering urine).

The membrane is thereafter put onto a pre-heated Columbia blood agar and incubated at 37°C for 6-8 hours.

The membrane is then recovered and put onto a glass fiber pad hydrated with DAPI solution staining at 5µg/mL or Hoechst 33342 solution at 5 µg/mL in Phosphate Buffer 0.05 M pH7.2 for 10 min at room temperature in the dark.

The membrane is then put onto a wash module containing Phosphate Buffer 0.05 M Tween 0.1% pH7.2. The washing is realized under the membrane through cycling of the wash buffer under the membrane.

Finally, the membrane is put onto a black palette hydrated with 3 drops of sterile water to allow correct adhesion of the membrane to the palette. The palette is then inserted into the optic device and the UV scan is initiated. It takes 7 min to scan the membrane.

Fluorescent spot appears onto the membrane (Figure 1).

DAPI is not toxic for microorganism, so the membrane can be replaced afterwards onto an agar plate and reincubated at 37°C to allow the growth of present microorganisms. After 24 h of total incubation, colonies appeared onto the membrane that permit to clearly compare the scan and the membrane and establish a comparison.

The DNA labeling obtained with Hoechst 33342 is similar to the staining with DAPI but less bright.

In comparable experimental conditions, using DAPI as fluorescent compound is more efficient than other fluorescent compounds labeling nucleic acids such Hoechst 33342.

### REFERENCES

**[1]** International application WO 2013/107759 A1 in the name of Centre National de la Recherche Scientifique, published on July 25, 2013.
**[2]** International application WO 2013/130875 A1 in the name of Harvard College, published on September 6, 2013.

## Claims

1. Method for detecting and optionally quantifying microorganisms in a liquid sample, comprising the steps of:
a) passing the liquid sample through a membrane thereby depositing the potentially present microorganisms on the upper surface of said membrane,
b) putting the lower surface of said membrane into contact with a culture medium and incubating the whole under conditions suitable for promoting the formation of microcolonies from the microorganisms,
c) putting the lower surface of said membrane into contact with a fluorescent compound capable of labeling an endogenous constituent of the microorganisms in order to label potentially formed microcolonies,
d) washing the lower surface of said membrane thereby eliminating the excess of fluorescent compound, and
e) detecting and optionally quantifying the potentially formed microcolonies labeled with the fluorescent compound, wherein the detection is performed using an optical device having a magnification between 1 and 50 and an optical resolution comprised between 6-20 Mpix.

2. Method according to claim 1, wherein said liquid sample is a biological fluid; a plant fluid such as sap, nectar and root exudate; a sample in a culture medium or in a biological culture reactor such as a cell culture of higher eukaryotes, of yeasts, of fungi or of algae; a liquid obtained from one (or more) animal or plant cell(s); a liquid sample obtained from an animal or plant tissue; a liquid sample obtained from a food matrix; a liquid sample from a chemical reactor; municipal, river, pond, lake, sea, or air-cooled tower water; a sample from a liquid industrial effluent; waste water coming in particular from intensive animal production or industries of the chemical, pharmaceutical, cosmetic or nuclear field; a liquid sample from a pharmaceutical product; a liquid sample from a cosmetic product; a fragrance; a soil sample or a mixture thereof.

3. Method according to claim 1 or 2, wherein said microorganisms are selected from yeasts, fungi, algae, bacteria of the Gram positive type, bacteria of the Gram negative type, archaea and mixtures thereof.

4. Method according to any one of claims 1 to 3, wherein said membrane is made of polytetrafluoroethylene (PTFE), polyester, polycarbonate, nylon, polyvinylidene fluoride (PVDF), cellulose and cellulose derivatives, such as cellulose acetate, cellulose nitrate, regenerated cellulose, nitrocellulose, cellophane, or mixed cellulose esters (MCE).

5. Method according to any one of claims 1 to 4, wherein said culture medium implemented at step (b) is a liquid culture medium.

6. Method according to any one of claims 1 to 4, wherein said culture medium implemented at step (b) is a solid culture medium.

7. Method according to any one of claims 1 to 6, wherein said fluorescent compound is capable to label mitochondria, cell membranes or nucleic acids.

8. Method according to any one of claims 1 to 7, wherein said fluorescent compound is 4',6-diamidino-2-phenylindole (DAPI).

9. Method according to any one of claims 1 to 8, wherein, during step (c), the lower surface of the membrane is brought into contact with a solution or dispersion of the fluorescent compound.

10. Method according to any one of claims 1 to 9, wherein said step (d) is performed by continuously flowing a rinsing solution at the level of the lower surface of the membrane.

11. Method according to any one of claims 1 to 10, wherein an excitation source implemented at said step (e) is light source optionally equipped with filters.

12. Method according to any one of claims 1 to 11, wherein said method is performed in less than 10 h.

13. Method according to anyone of claims 1 to 12, further comprising the steps of:
f) putting the lower surface of the membrane comprising at least a labeled microcolony, into contact with a culture medium and incubating the whole under conditions suitable for promoting the formation of colonies from the microcolonies in order to form at least one colony,
g) identifying the microorganism of the formed colony, and
h) submitting the formed colony to an antibiotic susceptibility testing.
